(19) Europäisches Patentamt
European Patent Office
Office européen des brevets

(11) Veröffentlichungsnummer: **0 081 207**
**A2**

(12) **EUROPÄISCHE PATENTANMELDUNG**

(21) Anmeldenummer: **82111161.4**

(22) Anmeldetag: **02.12.82**

(51) Int. Cl.[3]: **C 07 C 125/067**
C 07 C 155/08, C 07 C 127/19
C 07 C 103/365, C 07 C 103/38
C 07 C 103/737, C 07 D 295/20
C 07 C 87/52, C 07 C 97/10
C 07 C 91/40, A 01 N 47/20

(30) Priorität: **09.12.81 DE 3148595**

(43) Veröffentlichungstag der Anmeldung:
**15.06.83 Patentblatt 83/24**

(84) Benannte Vertragsstaaten:
**AT BE CH DE FR GB IT LI NL**

(71) Anmelder: **BASF Aktiengesellschaft**
**Carl-Bosch-Strasse 38**
**D-6700 Ludwigshafen(DE)**

(72) Erfinder: **Schirmer, Ulrich, Dr. Chem.**
**Berghalde 79**
**D-6900 Heidelberg(DE)**

(72) Erfinder: **Plath, Peter, Dr. Chem.**
**Berner Weg 24**
**D-6700 Ludwigshafen(DE)**

(72) Erfinder: **Reissenweber, Gernot, Dr. Chem.**
**Drosselstrasse 15**
**D-6737 Böhl-Iggelheim(DE)**

(72) Erfinder: **Wuerzer, Bruno, Dr. Dipl.-Landwirt**
**Ruedigerstrasse 13**
**D-6701 Otterstadt(DE)**

(72) Erfinder: **Retzlaff, Guenter, Dr.**
**Schillerstrasse 34**
**D-6725 Roemerberg(DE)**

(54) **Anilinderivate, Verfahren zu ihrer Herstellung und ihre Verwendung zur Bekämpfung unerwünschten Pflanzenwuchses.**

(57) Die Erfindung betrifft Anilinderivate der Formel

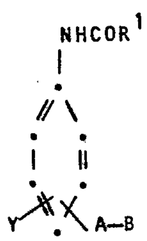

in der
$R^1$, Y, A und B die in der Beschreibung genannten Bedeutungen haben,
Verfahren zu ihrer Herstellung und ihre Verwendung zur Bekämpfung unerwünschten Pflanzenwuchses.

EP 0 081 207 A2

Croydon Printing Company Ltd

# Anilinderivate, Verfahren zu ihrer Herstellung und ihre Verwendung zur Bekämpfung unerwünschten Pflanzenwuchses

Die Erfindung betrifft Anilinderivate, Verfahren zu ihrer Herstellung, Herbizide, die diese Verbindungen als Wirkstoffe enthalten, sowie ein Verfahren zur Bekämpfung unerwünschten Pflanzenwuchses mit diesen Verbindungen.

Es ist bekannt, daß N-(3-tert.-Butylphenyl)-N'-methyl-N'-methoxy-harnstoff herbizid wirksam ist (DE-OS 27 39 349).

Es wurde gefunden, daß Anilinderivate der Formel

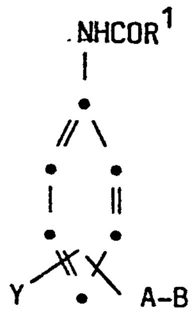

(I),

in der

$R^1$ Alkoxy, Alkylthio oder gegebenenfalls durch Halogen oder Alkoxy mit 1 bis 4 Kohlenstoffatomen substituiertes Alkyl mit jeweils bis zu 4 Kohlenstoffatomen oder Cycloalkyl mit 3 bis 6 Kohlenstoffatomen oder den Rest $-N{<}^{R^2}_{R^3}$, wobei $R^2$ und $R^3$ unabhängig voneinander Wasserstoff, Alkyl oder Alkoxy mit jeweils bis zu 4 Kohlenstoffatomen oder Cycloalkyl mit 3 bis 6 Kohlenstoffatomen oder $R^2$ und $R^3$ zusammen eine gegebenenfalls durch Methyl substituierte und gegebenenfalls Sauerstoff als Kettenglied enthaltende Alkylenkette mit bis zu 6 Kohlenstoffatomen bedeuten,

Y Wasserstoff, Halogen, Methyl, Methoxy oder Trifluormethyl,

H/P

A eine gegebenenfalls eine Ketogruppe oder eine sekundäre Carbinolgruppe als Kettenglied enthaltende, gegebenenfalls durch Methyl substituierte Alkylenkette mit 2 bis 9 Kohlenstoffatomen und

B unverzweigtes oder verzweigtes Alkyl mit bis zu 5 Kohlenstoffatomen, gegebenenfalls durch Alkyl mit 1 bis 4 Kohlenstoffatomen substituiertes Cycloalkyl mit bis zu 8 Kohlenstoffatomen, wobei bis zu 2 Ringglieder durch Sauerstoff ersetzt sein können, oder gegebenenfalls durch Alkyl mit 1 bis 4 Kohlenstoffatomen substituiertes Cycloalkenyl mit bis zu 6 Kohlenstoffatomen bedeuten und

A und B zusammen Reste mit mindestens 7 Kohlenstoffatomen bilden,

eine herbizide Wirkung haben und für Kulturpflanzen selektiv verträglich sind.

Die Substituenten in Formel I können die folgenden Bedeutungen haben:

$R^1$ bedeutet Alkoxy, Alkylthio oder gegebenenfalls durch Halogen oder Alkoxy mit 1 bis 4 Kohlenstoffatomen substituiertes Alkyl mit jeweils bis zu 4 Kohlenstoffatomen, beispielsweise Methoxy, Ethoxy, n-Propoxy, Isopropoxy, sec-Butoxy, tert.-Butoxy, Methylthio, Ethylthio, n-Propylthio, sec.-Butylthio, tert.-Butylthio, Methyl, Ethyl, n--Propyl, n-Butyl, sec.-Butyl, tert.-Butyl, Isobutyl, Isopropyl, Chlormethyl, 1,1-Dichlorethyl, Dichlormethyl, Methoxymethyl, 1-Methoxyethyl, 2-Methoxyethyl, Cycloalkyl mit 3 bis 6 Kohlenstoffatomen, beispielsweise Cyclopropyl, Cyclopentyl, Cyclohexyl, oder den Rest $-N\begin{smallmatrix}R^2\\R^3\end{smallmatrix}$, wobei $R^2$

und $R^3$ unabhängig voneinander Wasserstoff, Alkyl oder Alkoxy mit jeweils bis zu 4 Kohlenstoffatomen, beispielsweise Methyl, Ethyl, Isopropyl, n-Butyl, sec-Butyl, Isobutyl, tert.-Butyl, Methoxy, Ethoxy, oder Cycloalkyl mit 3 bis 6 Kohlenstoffatomen, beispielsweise Cyclopropyl, Cyclopentyl, Cyclohexyl, oder $R^2$ und $R^3$ zusammen eine gegebenenfalls durch Methyl substituierte und gegebenenfalls Sauerstoff als Kettenglied enthaltende Alkylenkette mit bis zu 6 Kohlenstoffatomen, beispielsweise Trimethylen, Tetramethylen, 1,4-Dimethyltetramethylen, Pentamethylen, Hexamethylen, 3-Oxapentamethylen, 1-Oxa-pentamethylen, 1-Oxa-tetramethylen bedeuten.

Y bedeutet Wasserstoff, Halogen, beispielsweise Fluor, Chlor, Brom, sowie Methyl, Methoxy oder Trifluormethyl.

A steht in meta- oder para-Stellung zu der Gruppe $-NH-CO-R^1$ und bedeutet eine gegebenenfalls eine Ketogruppe oder eine sekundäre Carbinolgruppe enthaltende, gegebenenfalls durch Methyl substituierte Alkylenkette mit 2 bis 9 Kohlenstoffatomen, beispielsweise eine $C_2$-Kette, wie $-(CH_2)_2-$, $-CO-CH_2-$ oder $-CH_2-CO-$, sowie die daraus durch Reduktion erhältlichen Carbinole $-CH(OH)-CH_2-$ bzw. $-CH_2-CH(OH)-$, eine $C_3$-Kette, wie $-(CH_2)_3-$, $-CO-(CH_2)_2-$, $-(CH_2)_2-CO-$, $-CH_2-CH(CH_3)-CH_2-$, $-CO-CH(CH_3)-CH_2-$, $-CH_2-CH(CH_3)-CO-$ sowie die daraus erhältlichen Carbinole, eine $C_4$-Kette, wie $-(CH_2)_4-$, $-CO-(CH_2)_3-$, $-(CH_2)_3-CO-$, $-(CH_2)_2-CH(CH_3)-CH_2-$, $-CH_2-CO-(CH_2)_2-$, $-(CH_2)_2-CO-(CH_2)-$, sowie die daraus erhältlichen Carbinole, eine $C_5$-Kette, wie $-(CH_2)_5-$, $-CO-(CH_2)_4-$, $-(CH_2)_4-CO-$, $-(CH_2)_2-CO-(CH_2)_2-$, $-CH_2-CH(CH_3)-(CH_2)_3-$, $-CO-(CH_2)_2-CH(CH_3)-CH_2-$, $-CH_2-CH(CH_3)-(CH_2)_2-CO-$, sowie die daraus erhältlichen Carbinole, eine $C_6$-Kette, wie $-(CH_2)_6-$, $-CO-(CH_2)_5-$, $-(CH_2)_5-CO-$, $-CH_2-CO-(CH_2)_4-$, $-(CH_2)_4-CO-CH_2-$, $-CH_2-CO-(CH_2)_2-CH(CH_3)-CH_2-$,

$-CH_2-CH(CH_3)-(CH_2)_2-CO-CH_2-$, $-(CH_2)_4-CH(CH_3)-CH_2-$, $-CH_2-CH(CH_3)-(CH_2)_4-$, sowie die daraus erhältlichen Carbinole, eine $C_7$-Kette, wie $-(CH_2)_7-$, $-CH_2-CH(CH_3)-(CH_2)_5-$, $-CO-(CH_2)_6-$, $-(CH_2)_6-CO-$, $-(CH_2)_3-CO-(CH_2)_3-$, $-(CH_2)_2-CO-(CH_2)_4-$, $-(CH_2)_4-CO-(CH_2)_2-$, $-CH_2-CH(CH_3)-(CH_2)_2-CO-(CH_2)_2-$, $-(CH_2)_2-CO-(CH_2)_2-CH(CH_3)-CH_2-$, sowie die daraus erhältlichen Carbinole, eine $C_8$-Kette, wie $-(CH_2)_8-$, $-CO-(CH_2)_7-$, $-(CH_2)_7-CO-$, sowie die daraus erhältlichen Carbinole oder eine $C_9$-Kette, wie $-(CH_2)_9-$, $-CO(CH_2)_8-$, $-(CH_2)_8-CO-$, $-(CH_2)_4-CO-(CH_2)_4-$, $-CH_2-CH(CH_3)-(CH_2)_2-CO-(CH_2)_4-$, $-(CH_2)_4-CO-(CH_2)_2-CH(CH_3)-CH_2-$, sowie die daraus erhältlichen Carbinole.

B steht für unverzweigtes oder verzweigtes Alkyl mit bis zu 5 Kohlenstoffatomen, wie Methyl, Ethyl, Isopropyl, t-Butyl, tert.-Amyl, für gegebenenfalls durch Alkyl mit 1 bis 4 Kohlenstoffatomen substituiertes Cycloalkyl mit bis zu 8 Kohlenstoffatomen, wobei bis zu 2 Ringglieder durch Sauerstoff ersetzt sein können, wie Cyclopentyl, Cyclohexyl, 3-Methylcyclohexyl, 4-Methylcyclohexyl, 4-tert.-Butyl-cyclohexyl, Cycloheptyl, Cyclooctyl, 1-Methyl-4-ethyl-3,5-dioxacyclohexyl, 4-Isopropyl-3,5-dioxacyclohexyl, 2-Oxacyclohexyl, oder für gegebenenfalls durch Alkyl mit 1 bis 4 Kohlenstoffatomen substituiertes Cycloalkenyl mit bis zu 6 Kohlenstoffatomen, wie 4-Methyl-cyclohexen-3-yl, 3,4-Dimethyl-cyclohexen-3-yl.

Bevorzugte Verbindungen der Formel I sind solche, bei denen $R^1$ für Alkylthio oder Alkyl mit 1 bis 4 Kohlenstoffatomen, vorzugsweise Methylthio oder Ethyl, Cycloalkyl mit 3 bis 6 Kohlenstoffatomen, vorzugsweise Cyclopropyl, oder den Rest $-N{<}^{R^2}_{R^3}$ , wobei $R^2$ und $R^3$ unabhängig voneinander

Wasserstoff oder Alkyl oder Alkoxy mit 1 bis 3 Kohlenstoffatomen, vorzugsweise Methoxy, Methyl, bedeuten, Y für Wasserstoff, A für eine gegebenenfalls eine Ketogruppe als Kettenglied enthaltende, gegebenenfalls durch Methyl substituierte Alkylenkette mit 2 bis 4 Kohlenstoffatomen und B für verzweigtes Alkyl mit 3 bis 5 Kohlenstoffatomen, für gegebenenfalls durch Alkyl mit 1 bis 3 Kohlenstoffatomen substituiertes Cycloalkyl mit 5 oder 6 Kohlenstoffatomen, wobei 2 Ringglieder durch Sauerstofff ersetzt sein können, oder für methylsubstituiertes Cyclohexenyl stehen und
A und B zusammen Reste mit mindestens 7 Kohlenstoffatomen bilden.

Man erhält die Anilinderivate der Formel I durch Umsetzung von Aminen der Formel

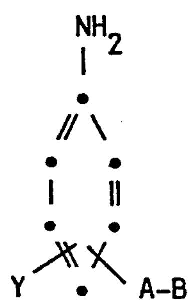

(II),

in der
Y, A und B die obengenannten Bedeutungen haben, mit Verbindungen der Formel

$$R^1 - CO - X \qquad (III),$$

in der $R^1$ die obengenannten Bedeutungen hat und X eine Abgangsgruppe, vorzugsweise Halogen, wie Chlor oder Brom, oder $R^1$-CO-O- bedeutet.

Die Umsetzung wird in einem inerten organischen Lösungsmittel durchgeführt. Geeignet sind Ether, wie Tetrahydrofuran, Dimethoxyethan, Diethylether, Methyl-tert.-butyl-ether, Ester wie Essigsäureethylester, gegebenenfalls chlorierte aliphatische oder aromatische Kohlenwasserstoffe, wie Toluol, Dichlormethan, Dichlorethan, Chlorbenzol oder Pyridin. Auch Gemische dieser Lösungsmittel können verwendet werden. Die Menge an Lösungsmittel, bezogen auf Amin der Formel II beträgt 100 bis 5000 Gew.%.

Zweckmäßigerweise wird die Umsetzung in Gegenwart eines Säureakzeptors durchgeführt. In Betracht kommen Alkalihydroxide, Alkalicarbonate, Alkalihydrogencarbonate, Erdalkalihydroxide, Erdalkalicarbonate, Erdalkalihydrogencarbonate, Erdalkalioxide oder Amine, beispielsweise Natriumhydrogencarbonat, Kaliumcarbonat, Triethylamin, Pyridin, N,N-Dimethylanilin, N,N-Dimethyl-N-cyclohexylamin, Chinolin. Die Menge an Säureakzeptor beträgt 1 bis 4 Mol, bezogen auf 1 Mol Verbindung der Formel III.

Die Ausgangsstoffe der Formeln II und III werden im Verhältnis 1 : 1 bis 1 : 2 Mol miteinander umgesetzt. Die Reaktionstemperatur liegt zwischen 0 und 80°C, vorzugsweise zwischen 20 und 40°C.

Die Amine der Formel II sind neu. Sie werden nach bekannten Verfahren durch Aldolkondensation und anschließende Hydrierung erhalten (Organic Reactions, Bd. 16, S. 1 ff (1968)). Man setzt beispielsweise Nitrobenzaldehyde der Formel V mit Verbindungen der Formel VI um:

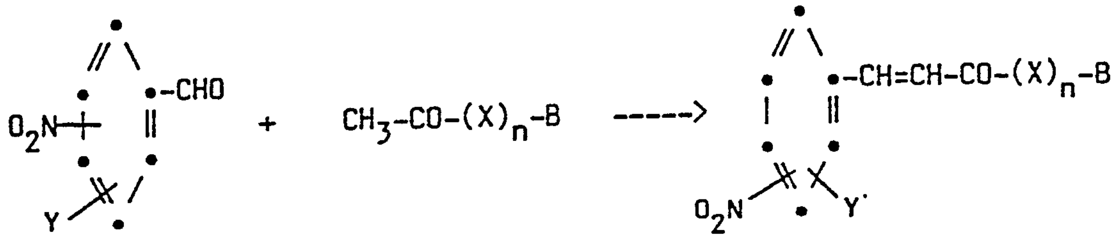

(V) (VI) (VII)

In diesen Formeln haben Y und B die oben genannten Bedeutungen, X steht für Methylen oder Vinylen und n bedeutet 0, 1 oder 2.

Außerdem können Nitrozimtaldehyde der Formel VIII, die gegebenenfalls in Nachbarstellung zur Doppelbindung durch Alkyl substituiert sind, mit Verbindungen der Formel VI zu Verbindungen der Formel IX umgesetzt werden:

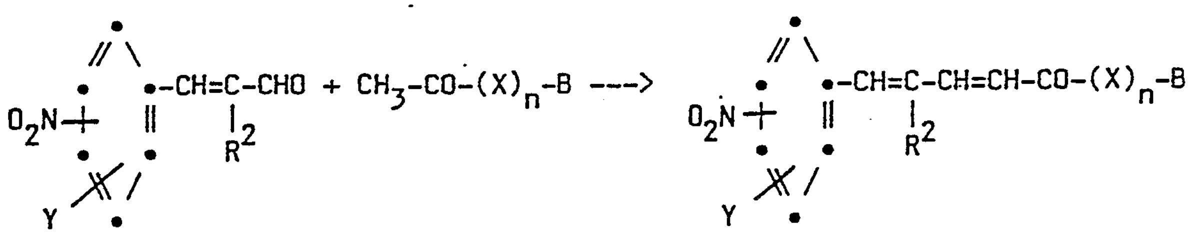

(VIII) (VI) (IX)

Dabei haben B, X, Y und n die obengenannten Bedeutungen, $R^2$ steht für Alkyl mit 1 bis 4 Kohlenstoffatomen.

Setzt man Nitroacetophenone mit Aldehyden um, erhält man Verbindungen der Formel X:

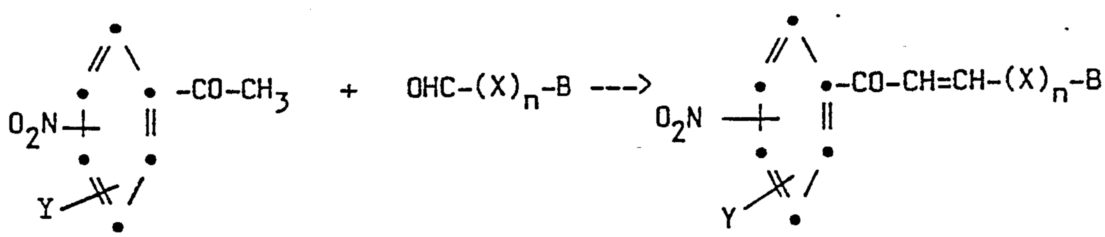

(X)

Durch katalytische Reduktion der Nitroverbindungen der Formeln VII, IX und X lassen sich die Amine der Formel II, in der A eine eine Ketogruppe oder eine sekundäre Carbinolgruppe als Kettenglied enthaltende, gegebenenfalls durch Methyl substituierte Alkylenkette mit 3 bis 9 Kohlenstoffatomen bedeutet und Y und B die oben genannten Bedeutungen haben, herstellen.

Die Hydrierung erfolgt in an sich üblicher Weise in einem organischen Lösungsmittel, wie Methanol, Tetrahydrofuran, Eisessig oder Essigsäureethylester, in Gegenwart von Palladium auf Aktivkohle oder in Gegenwart von Raney-Nickel. Vorzugsweise wird Palladium auf Kohle verwendet.

Die Hydrierung zum gesättigten Anilinoketon erfolgt bereits bei Temperaturen von 0 bis 30°C, vorzugsweise bei 25°C, und bei einem Druck von 1,01 bis 20 bar, bevorzugt bei 1,1 bar. Verlängerung der Reaktionsdauer, Erhöhung der Hydriertemperatur und/oder Druckerhöhung bis zu 50 bar führt zur Reduktion der Ketogruppe zur Carbinolgruppe. (Houben-Weyl, Methoden der Org. Chemie, Bd. 4/1c, S. 13ff, Georg-Thieme-Verlag, Stuttgart, 1980.).

Die Wasserstoffaufnahme bei der Hydrierung der vorgenannten gesättigten oder ungesättigten Nitrophenylketone erfolgt stufenweise und kann entweder nach Erreichen der gesättigten Anilinoketon-Stufe abgebrochen werden oder bis zur Anilinocarbinolstufe weitergeführt werden.

Amine der Formel II, in der A eine gegebenenfalls durch Methyl substituierte Alkylenkette mit 2 bis 9 Kohlenstoffatomen bedeutet, erhält man zweckmäßigerweise durch Wolff--Kishner-Reduktion von Aminen der Formel II, in der A eine eine Ketogruppe als Kettenglied enthaltende, gegebenenfalls durch Methyl substituierte Alkylenkette mit 2 bis

9 Kohlenstoffatomen bedeutet (Organicum, VEB Deutscher Verlag der Wissenschaften, Berlin, 1967, S. 426 ff.).

Die folgenden Beispiele erläutern die Synthese der Amine der Formel II:

Beispiel A

1-(3-Amino-phenyl)-4,4-dimethyl-n-pentan-3-on

Zu einer Mischung aus 302 g 3-Nitrobenzaldehyd und 200 g 3,3-Dimethylbutanon-2 in 2 l Methanol tropft man innerhalb von 6 Stunden eine Lösung von 80 g NaOH in 600 ml Wasser hinzu. Nach 12-stündigem Nachrühren wird abgesaugt und mit Methanol und Wasser nachgewaschen. Man erhält 314 g 1-(3-Nitrophenyl)-4,4-dimethyl-n-pent-1-en-3-on vom Fp. 86-89°C.

Eine Mischung aus 210 g 1-(3-Nitrophenyl)-4,4-dimethyl--n-pent-1-en-3-on, 10 g 10 % Pd/Tierkohle und 2 l Tetrahydrofuran wird bei einem Wasserstoffdruck von 1,1 bar und einer Temperatur von etwa 20 bis 30°C hydriert. Nach Aufnahme von 81 l Wasserstoff wird die Hydrierung abgebrochen. Das Gemisch wird über $Na_2SO_4$ getrocknet, filtriert, eingeengt und destilliert. Man erhält 165 g 1-(3-Amino-phenyl)-4,4-dimethyl-n-pentan-3-on vom $Kp_{0,15}$ = 133 bis 136°C.

Beispiel B

1-(3-Amino-phenyl)-4,4-dimethyl-n-pentan

Zu einer Mischung aus 69 g 1-(3-Amino-phenyl)-4,4-dimethyl--n-pentan-3-on, 76 g gepulvertem KOH und 300 ml Triethylenglykol tropft man 51 g Hydrazinhydrat und erwärmt unter gutem Rühren 2 Stunden lang auf 120 bis 130°C. Dann steigert man die Temperatur langsam auf etwa 190°C, wobei

Wasser und überschüssiges Hydrazinhydrat abdestilliert werden. Man hält etwa 1 Stunde bei 190°C, läßt abkühlen, schüttelt mit Ether aus, trocknet die Etherphase über $Na_2SO_4$, filtriert, engt ein und destilliert den Rückstand. Man erhält 48 g 1-(3-Amino-phenyl)-4,4-dimethyl-n-pentan vom $Kp_{0,2}$ = 96 bis 99°C.

Entsprechend lassen sich folgende Amine der Formel II herstellen:

| Stellung von -A-B | A | B | Y | Kp |
|---|---|---|---|---|
| 3 | $-(CH_2)_3-$ | t.-Butyl | H | 110°C/0,3 mbar |
| 4 | $-(CH_2)_3-$ | " | H | 97°C/0,4 mbar |
| 3 | $-(CH_2)_4-$ | " | H | |
| 4 | $-(CH_2)_4-$ | " | H | 96-98°C/0,1 mbar |
| 3 | $-(CH_2)_5-$ | " | H | |
| 4 | $-(CH_2)_5)-$ | " | H | |
| 3 | $-(CH_2)_2CO-$ | " | H | 132-134°C/ 0,1 mbar |
| 4 | $-(CH_2)_2CO-$ | " | H | 128°C/0,3 mbar |
| 3 | $-(CH_2)_2CH(OH)-$ | " | H | 138°C/0,3 mbar |
| 4 | $-(CH_2)_2CH(OH)-$ | " | H | 125°C/0,4 mbar |
| 3 | $-(CH_2)_2-COCH_2-$ | " | H | Öl |
| 4 | $-(CH_2)_2-COCH_2-$ | " | H | Öl |
| 3 | $-(CH_2)_2-CH(OH)-CH_2-$ | " | H | |
| 4 | $-(CH_2)_2-CH(OH)-CH_2-$ | " | H | |
| 3 | $-(CH_2)_2COCH_2-$ | i-Propyl | H | Öl |
| 3 | $-(CH_2)_4-$ | " | H | 105°C/1 mbar |
| 3 | $-(CH_2)_2-CH(OH)-CH_2-$ | " | H | |
| 4 | $-CH_2CH(CH_3)CH_2CH_2CO-$ | t-Butyl | H | |
| 4 | $-CH_2CH(CH_3)CH_2CH_2-CH(OH)-$ | " | H | |
| 4 | $-CH_2CH(CH_3)-(CH_2)_3-$ | " | H | |

| Stellung von -A-B | A | B | Y | Kp |
|---|---|---|---|---|
| 4 | $-(CH_2)_2CO-$ | 4-Methylcyclohexen-3-yl | H | Öl |
| 4 | $-(CH_2)_3-$ | " | H | 168-180°C/ 2 mbar |
| 3 | $-(CH_2)_2CO-$ | 1-Methyl-4-ethyl-3,5-dioxacyclohexyl | H | Öl |
| 3 | $-(CH_2)_3-$ | " | H | 158°C/0,1 mbar |
| 4 | $-(CH_2)_2CO-$ | t.-Butyl | 3-Cl | |
| 4 | $-(CH_2)_3-$ | " | 3-Cl | |
| 4 | $-(CH_2)_2CO-$ | " | $3-CH_3$ | |
| 4 | $-(CH_2)_2CO-$ | " | 3-F | |
| 4 | $-(CH_2)_2CO-$ | " | $3-CF_3$ | |
| 3 | $-(CH_2)_3-$ | " | $4-OCH_3$ | |
| 4 | $-(CH_2)_2CO(CH_2)_2-$ | " | H | |
| 3 | $-(CH_2)_6-$ | " | H | |
| 3 | $-(CH_2)_7-$ | " | H | |
| 4 | $-(CH_2)_8-$ | " | H | |
| 4 | $-(CH_2)_9-$ | " | H | |
| 3 | $-(CH_2)_3-$ | Cyclohexyl | H | |
| 3 | $-(CH_2)_2CO-$ | " | H | |

| Stellung von -A-B | A | B | Y | Kp |
|---|---|---|---|---|
| 4 | $-(CH_2)_3-$ | Cyclohexyl | H | |
| 4 | $-(CH_2)_2CO-$ | " | | |
| 4 | $-(CH_2)_4CO(CH_2)_2-$ | t.-Butyl | H | |
| 3 | $-CO(CH_2)_2CH(CH_3)CH_2-$ | Cyclohexyl | H | |
| 4 | $-CO(CH_2)_2CH(CH_3)CH_2-$ | " | H | |
| 3 | $-(CH_2)_3CH(CH_3)CH_2-$ | " | H | |
| 4 | $-(CH_2)_3CH(CH_3)CH_2-$ | " | H | |
| 3 | $-(CH_2)_3-$ | sec.-Butyl | H | |
| 4 | $-(CH_2)_3-$ | " | | |

Anilinderivate der Formel I, bei denen $R^1$ Alkoxy oder Alkylthio oder den Rest $-N{<}^{R^2}_{R^3}$ und A eine gegebenenfalls eine Ketogruppe als Kettenglied enthaltende, gegebenenfalls durch Methyl substituierte Alkylenkette mit 2 bis 9 Kohlenstoffatomen bedeuten und Y und B die obengenannten Bedeutungen haben, erhält man durch Umsetzung von Isocyanaten der Formel

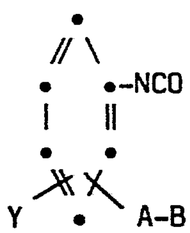

(IV),

in der Y, A und B die oben genannten Bedeutungen haben, mit einer Verbindung der Formel $R^1H$, in der $R^1$ die oben genannten Bedeutungen hat, bei einer Temperatur zwischen 0 und 150, vorzugsweise zwischen 0 und 30°C.

Die Umsetzung wird in einem unter den Reaktionsbedingungen inerten Lösungsmittel durchgeführt. Geeignet sind Ether, wie Diethylether, Methyl-tert.-butyl-ether, Tetrahydrofuran, Ester, wie Essigsäureethylester, gegebenenfalls chlorierte aliphatische oder aromatische Kohlenwasserstoffe, wie Ligroin, Toluol, Cyclohexan, Benzin, Dichlormethan, Chloroform, Chlorbenzol, o-, m-, p-Dichlorbenzol, Nitrobenzol, Ketone, wie Aceton, Nitrile, wie Acetonitril, oder Dimethylformamid. Auch Gemische dieser Lösungsmittel können verwendet werden.

Die Umsetzung der Isocyanate der Formel IV mit Alkoholen, Mercaptanen oder Aminen der Formel $R^1H$ kann gegebenenfalls durch Zugabe eines für Isocyanatreaktionen gebräuchlichen Katalysators beschleunigt werden, z.B. durch tert.-

-Amine, wie Triethylamin, 1,4-Diaza-bicyclo[2.2.2]octan, Stickstoffheterocyclen, wie Pyridin oder 1,2-Dimethylimidazol oder organische Zinnverbindungen, wie Dibutylzinndiacetat, Dimethylzinndichlorid.

Zur Herstellung der Isocyanate gemäß Formel IV setzt man die Amine der Formel II mit Phosgen um (Liebigs Ann.Chem. 562, 75ff, (1949)).

Die folgenden Beispiele erläutern die Herstellung der Anilinderivate der Formel I:

Beispiel 1
N-[3-(4,4-Dimethyl-n-pentyl)-phenyl]-N'-methyl-N'-methoxy-harnstoff

Zu einer Mischung aus 15 g 3-(4,4-Dimethyl-n-pentyl)-anilin, 8 g Natriumhydrogencarbonat und 200 ml Tetrahydrofuran tropft man bei Raumtemperatur unter Rühren 9,7 g N-Methoxy-N-methyl-carbaminsäurechlorid. Nach 12 stündigem Rühren wird filtriert, das Filtrat eingeengt und der verbleibende Rückstand mit Petrolether verrieben und abgesaugt. Man erhält 18,4 g N-[3-(4,4-Dimethyl-n-pentyl)-phenyl]-N'-methyl-N'-methoxy-harnstoff vom Fp. 66 bis 68°C.

Beispiel 2
N-[3-(4,4-Dimethyl-n-pentyl)-phenyl]-N',N'-dimethyl-harnstoff

Bei -10°C werden 190 g Phosgen in 200 ml Toluol vorgelegt. Ebenfalls bei -10°C tropft man unter gutem Rühren eine Mischung aus 146 g 3-(4,4-Dimethyl-n-pentyl)-anilin in 250 ml Toluol zu, man erwärmt langsam bis 110°C und leitet etwa 12 Stunden lang Phosgen durch die Mischung. Dann wird eine Stunde lang Stickstoff durchgeleitet und anschließend

destilliert. Man erhält 142 g 3-(4,4-Dimethyl-n-pentyl)--phenyl-isocyanat vom Kp. = 118°C/0,7 mbar.

14 g 3-(4,4-Dimethyl-n-pentyl)-phenyl-isocyanat werden bei 0°C langsam zu einer Lösung von 1,3 g Dimethylamin in 200 ml Toluol getropft. Nach 12 stündigem Rühren wird eingeengt, und der entstandene Kristallbrei wird mit Petrolether verrührt und abgesaugt. Man erhält 14,8 g N-[3-(4,4-Dimethyl-n-pentyl)-phenyl]-N',N'-dimethyl-harnstoff vom Fp. 150°C bis 151°C.

Analog werden beispielsweise die folgenden Verbindungen der Formel I synthetisiert:

| Nr. | $R^1$ | Stellung von -A-B | A | B | Y | Fp [°C] |
|---|---|---|---|---|---|---|
| 1 | $OCH_3$ | 3 | $-(CH_2)_3-$ | t-Butyl | H | 65-67 |
| 2 | $SCH_3$ | 3 | $-(CH_2)_3-$ | " | H | 68-70 |
| 3 | $O-t-C_4H_9$ | 3 | $-(CH_2)_3-$ | " | H | 117-119 |
| 4 | $C_2H_5$ | 3 | $-(CH_2)_3-$ | " | H | 90-92 |
| 5 | Cyclopropyl | 3 | $-(CH_2)_3-$ | " | H | 114-116 |
| 6 | $t-C_4H_9$ | 3 | $-(CH_2)_3-$ | " | H | 109-110 |
| 7 | $NHCH_3$ | 3 | $-(CH_2)_3-$ | " | H | 109-110 |
| 8 | $N(CH_3)_2$ | 3 | $-(CH_2)_3-$ | " | H | 150-151 |
| 9 | $N(OCH_3)CH_3$ | 3 | $-(CH_2)_3-$ | " | H | 66-68 |
| 10 | 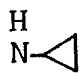 | 3 | $-(CH_2)_3-$ | " | H | 107-109 |
| 11 | 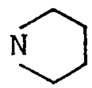 | 3 | $-(CH_2)_3-$ | " | H | 127-129 |
| 12 | $N(OCH_3)CH_3$ | 3 | $-(CH_2)_5-$ | " | H | |
| 13 | " | 4 | $-(CH_2)_3-$ | " | H | 77-79 |
| 14 | " | 3 | $-(CH_2)_4$ | " | H | 65-67 |
| 15 | " | 4 | $-(CH_2)_4$ | " | H | |
| 16 | " | 3 | $-(CH_2)_3-$ | " | 4-Br | |
| 17 | " | 3 | $-(CH_2)_3-$ | " | 4-Cl | |
| 18 | " | 3 | $-(CH_2)_3-$ | " | $4-CH_3$ | |
| 19 | " | 4 | $-(CH_2)_3-$ | " | $3-CH_3$ | |
| 20 | " | 4 | $-(CH_2)_3-$ | " | 3-Cl | |

| Nr. | $R^1$ | Stellung von -A-B | A | B | Y | Fp [°C] |
|---|---|---|---|---|---|---|
| 21 | $N(OCH_3)CH_3$ | 4 | $-(CH_2)_3-$ | t-Butyl | $3-CF_3$ | |
| 22 | " | 3 | $-(CH_2)_3-$ | t-Butyl | $4-OCH_3$ | |
| 23 | " | 3 | $-(CH_2)_4-$ | i-Propyl | H | 47-48 |
| 24 | " | 4 | $-(CH_2)_5-$ | t-Butyl | H | |
| 25 | $SCH_3$ | 3 | $-CH_2CH_2CO-$ | t-Butyl | H | 70-72 |
| 26 | $OCH_3$ | 3 | $-CH_2CH_2CO-$ | " | H | 49-51 |
| 27 | $N(OCH_3)CH_3$ | 3 | $-CH_2CH_2CO-$ | " | H | 102-105 |
| 28 | $OCH_3$ | 3 | $-CH_2CH_2CH(OH)-$ | " | H | 86-88 |
| 29 | $SCH_3$ | 3 | $-CH_2CH_2CH(OH)-$ | " | H | 73-75 |
| 30 | $N(OCH_3)CH_3$ | 3 | $-CH_2CH_2CH(OH)-$ | " | H | |
| 31 | $N(OCH_3)CH_3$ | 3 | $-CH_2CH_2-CO-CH_2-$ | " | H | |
| 32 | $N(OCH_3)CH_3$ | 4 | $-CH_2CH_2COCH_2-$ | " | H | 56-57 |
| 33 | $C_2H_5$ | 4 | $-CH_2CH_2COCH_2-$ | " | H | |
| 34 | $C_2H_5$ | 3 | $-(CH_2)_4-$ | " | H | 61-63 |
| 35 | $OCH_3$ | 3 | $-(CH_2)_4-$ | " | H | 47-50 |
| 36 | $SCH_3$ | 3 | $-(CH_2)_4-$ | " | H | 52-55 |
| 37 | $NHCH_3$ | 3 | $-(CH_2)_4-$ | " | H | 105-107 |
| 38 | $OCH_3$ | 3 | $-(CH_2)_4-$ | i-Propyl | H | Öl |
| 39 | $N(CH_3)_2$ | 3 | $-(CH_2)_4-$ | i-Propyl | H | |
| 40 | $SCH_3$ | 3 | $-(CH_2)_4-$ | i-Propyl | H | 67-69 |

| Nr. | $R^1$ | Stellung von -A-B | A | B | Y | Fp [°C] |
|---|---|---|---|---|---|---|
| 41 | $C_2H_5$ | 3 | $-(CH_2)_4-$ | i-Propyl | H | 33-37 |
| 42 | $N(OCH_3)CH_3$ | 4 | $-(CH_2)_2CO(CH_2)_2-$ | t-Butyl | H | |
| 43 | $N(OCH_3)CH_3$ | 3 | $-(CH_2)_3-$ | 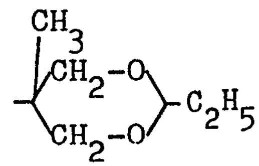 | H | Öl |
| 44 | $SCH_3$ | 3 | $-(CH_2)_3-$ | " | H | Öl |
| 45 | $OCH_3$ | 3 | $-(CH_2)_3-$ | " | H | Öl |
| 46 | $C_2H_5$ | 3 | $-(CH_2)_3-$ | " | H | Öl |
| 47 | $N(OCH_3)CH_3$ | 4 | $-(CH_2)_3-$ | " | H | |
| 48 | $N(OCH_3)CH_3$ | 4 | $-(CH_2)_3-$ | 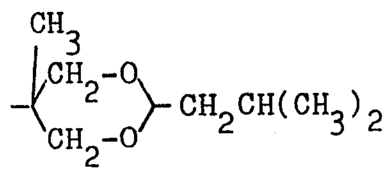 | H | |
| 49 | $N(OCH_3)CH_3$ | 4 | $-(CH_2)_3-$ | 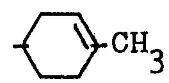 | H | 60-61 |
| 50 | $C_2H_5$ | 4 | $-(CH_2)_3-$ | " | H | 94-96 |
| 51 | $C_2H_5$ | 4 | $-CH_2CH_2CO-$ | " | H | 117-120 |
| 52 | $N(OCH_3)CH_3$ | 4 | $-CH_2CH_2CO-$ | " | H | 67-68 |
| 53 | $N(OCH_3)CH_3$ | 4 | $-(CH_2)_2CO-$ | t-Butyl | 3-Cl | |
| 54 | $N(CH_3)_2$ | 4 | $-(CH_2)_2CO-$ | t-Butyl | $3-CF_3$ | |

| Nr. | $R^1$ | Stellung von -A-B | A | B | Y | Fp [°C] |
|---|---|---|---|---|---|---|
| 55 | $N(OCH_3)CH_3$ | 4 | $-CH_2CH(CH_3)(CH_2)_2CO-$ | t-Butyl | H | Öl |
| 56 | $C_2H_5$ | 4 | $-CH_2CH(CH_3)(CH_2)_2CO-$ | t-Butyl | H | 81-83 |
| 57 | $N(OCH_3)CH_3$ | 4 | $-(CH_2)_2CH(OH)-CH_2$ | t-Butyl | H | |
| 58 | $N(OCH_3)CH_3$ | 4 | $-(CH_2)_2CH(OH)-$ | t-Butyl | H | 68-70 |
| 59 | $N(OCH_3)CH_3$ | 4 | $-(CH_2)_2CO-$ | Cyclohexyl | H | |
| 60 | $N(OCH_3)CH_3$ | 4 | $-CH_2CH(CH_3)(CH_2)_3-$ | t-Butyl | H | |
| 61 | $N(OCH_3)CH_3$ | 3 | $-CH_2CH(CH_3)(CH_2)_3-$ | t-Butyl | H | |
| 62 | $N(OCH_3)CH_3$ | 4 | $-CO(CH_2)_2-$ | t-Butyl | H | |
| 63 | $N(OCH_3)CH_3$ | 3 | $-CO(CH_2)_2-$ | t-Butyl | H | |
| 64 | $N(OCH_3)CH_3$ | 3 | $-CO(CH_2)_2-$ | Cyclohexyl | H | |
| 65 | $N(OC_2H_5)CH_3$ | 4 | $-(CH_2)_2CO-$ | t-Butyl | H | |
| 66 | 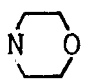 | 4 | $-(CH_2)_2CO-$ | " | H | |
| 67 | 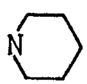 | 4 | $-(CH_2)_2CO-$ | " | H | |
| 68 | 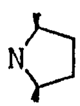 | 4 | $-(CH_2)_2CO-$ | " | H | |
| 69 | 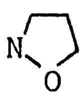 | 4 | $-(CH_2)_2CO-$ | " | H | |

| Nr. | $R^1$ | Stellung von -A-B | A | B | Y | Fp [°C] |
|---|---|---|---|---|---|---|
| 70 | 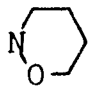 | 4 | $-(CH_2)_2CO-$ | t-Butyl | H | |
| 71 | $OC_2H_5$ | 4 | $-(CH_2)_2CO-$ | t-Butyl | H | |
| 72 | $CH(CH_3)C_2H_5$ | 4 | $-(CH_2)_2CO-$ | t-Butyl | H | |
| 73 | $N(C_2H_5)_2$ | 4 | $-(CH_2)_2CO-$ | t-Butyl | H | |
| 74 | $N(OC_2H_5)C_2H_5$ | 4 | $-(CH_2)_2CO-$ | t-Butyl | H | |
| 75 | $N(OCH_3)CH_3$ | 3 | $-(CH_2)_3-$ | Cyclohexyl | H | |
| 76 | $N(OCH_3)CH_3$ | 4 | $-(CH_2)_3-$ | Cyclohexyl | H | |
| 77 | $N(OCH_3)CH_3$ | 4 | $-CH_2CH_2CO-$ | $t-C_5H_{11}$ | H | |
| 78 | $N(OCH_3)CH_3$ | 4 | $-CH_2CH_2CO-$ | $4-t-C_4H_9$--cyclohexyl | H | |
| 79 | $N(OCH_3)CH_3$ | 4 | $-CH_2CH_2CO-$ | $4-CH_3$-cyclohexyl | H | |
| 80 | $N(OCH_3)CH_3$ | 3 | $-(CH_2)_3-$ | $4-CH_3$-cyclohexyl | H | |
| 81 | $C_2H_5$ | 3 | $-(CH_2)_3-$ | $4-CH_3$-cyclohexyl | H | |
| 82 | $SCH_3$ | 3 | $-(CH_2)_3-$ | $4-CH_3$-cyclohexyl | H | |
| 83 | $N(OCH_3)CH_3$ | 4 | $-(CH_2)_3-$ | $4-CH_3$-cyclohexyl | H | |

| Nr. | $R^1$ | Stellung von -A-B | A | B | Y | Fp [°C] |
|---|---|---|---|---|---|---|
| 84 | $C_2H_5$ | 4 | $-(CH_2)_3-$ | 4-$CH_3$-cyclohexyl | H | |
| 85 | $N(OCH_3)CH_3$ | 3 | $-(CH_2)_3-$ | sec.-Butyl | H | |
| 86 | $N(OCH_3)CH_3$ | 4 | $-(CH_2)_3-$ | sec.-Butyl | H | |
| 87 | $C_2H_5$ | 3 | $-(CH_2)_3CH(CH_3)CH_2-$ | Cyclohexyl | H | |
| 88 | $SCH_3$ | 3 | $-(CH_2)_3CH(CH_3)CH_2-$ | Cyclohexyl | H | |
| 89 | $N(OCH_3)CH_3$ | 3 | $-(CH_2)_3CH(CH_3)CH_2-$ | Cyclohexyl | H | |
| 90 | $N(OCH_3)CH_3$ | 4 | $-(CH_2)_3CH(CH_3)CH_2-$ | Cyclohexyl | H | |
| 91 | $SCH_3$ | 4 | $-(CH_2)_2CO-$ | t-Butyl | H | 117-118 |
| 92 | $OCH_3$ | 4 | $-(CH_2)_2CO-$ | t-Butyl | H | 78-79 |
| 93 | $C_2H_5$ | 4 | $-(CH_2)_2CO-$ | t-Butyl | H | 103-105 |
| 94 | $N(OCH_3)CH_3$ | 4 | $-(CH_2)_2CO-$ | t-Butyl | H | 74-76 |
| 95 | $OCH_3$ | 4 | $-(CH_2)_3-$ | t-Butyl | H | 66-67 |
| 96 | $SCH_3$ | 4 | $-(CH_2)_3-$ | t-Butyl | H | 90-91 |
| 97 | $NHCH_3$ | 4 | $-(CH_2)_3-$ | t-Butyl | H | 156-157 |
| 98 | $C_2H_5$ | 4 | $-(CH_2)_3-$ | t-Butyl | H | 93-95 |
| 99 | $C_2H_5$ | 3 | $-(CH_2)_3-$ | 3-$CH_3$-cyclohexyl | H | |

| Nr. | $R^1$ | Stellung von -A-B | A | B | Y | Fp [°C] |
|---|---|---|---|---|---|---|
| 100 | $C_2H_5$ | 3 | $-(CH_2)_3-$ | Cycloheptyl | H | |
| 101 | $C_2H_5$ | 3 | $-(CH_2)_3-$ | Cyclopentyl | H | |
| 102 | $C_2H_5$ | 3 | $-(CH_2)_8-$ | t-Butyl | H | |
| 103 | $C_2H_5$ | 3 | $-(CH_2)_9-$ | t-Butyl | H | |
| 104 | $C_2H_5$ | 3 | $-(CH_2)_7-$ | t-Butyl | H | |
| 105 | $C_2H_5$ | 3 | $-(CH_2)_4CO-$ | t-Butyl | H | |
| 106 | $N(OCH_3)CH_3$ | 4 | $-(CH_2)_4CO-$ | t-Butyl | H | |
| 107 | $CH_2Cl$ | 4 | $-(CH_2)_3-$ | t-Butyl | H | 101-104 |
| 108 | $N(OC_2H_5)CH_3$ | 4 | $-(CH_2)_3-$ | t-Butyl | H | Öl |
| 109 | $C_2H_5$ | 3 | $-CH(OH)-(CH_2)_2-$ | t-Butyl | H | 105-107 |
| 110 | $N(OCH_3)CH_3$ | 3 | $-CH(OH)-(CH_2)_2-$ | t-Butyl | H | 96-98 |

Die Verbindungen der Formel I oder deren Säureadditionssalze bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende

Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat,

Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

I. Man vermischt 90 Gewichtsteile der Verbindung Nr. 4 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 14 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung Nr. 5 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteile des Anlagerungsproduktes von 40 Mol Ethylenoxid an 1 Mol Ricinusöl besteht. Durch Eingießen und feines Verteilen der Lösung in 100 000 Gewichtsteilen Wasser

erhält man eine wäßrige Dispersion, die 0,02 Gewichtsprozent des Wirkstoffs enthält.

IV. 20 Gewichtsteile der Verbindung Nr. 27 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 20 Gewichtsteile der Verbindung Nr. 43 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile der Verbindung Nr. 94 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile der Verbindung Nr. 2 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile der Verbindung Nr. 23 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation der Wirkstoffe bzw. der Mittel kann im Vorauflaufverfahren oder im Nachauflaufverfahren erfolgen. Vorzugsweise werden die neuen Wirkstoffe nach dem Auflaufen der unerwünschten Pflanzen, sowohl auf Kulturflächen als auch auf unbebautem Land, ausgebracht. Sind die Wirkstoffe für die Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Bekämpfungsziel und Wachstumsstadium 0,05 bis 10 kg/ha und mehr, vorzugsweise 0,1 bis 2,5 kg/ha.

Die herbizide Wirkung von Verbindungen der Formel I wird durch Gewächshausversuche gezeigt:

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt flach eingesät. Unmittelbar danach erfolgt bei Vorauflaufbehandlung das Aufbringen der Wirkstoffe auf die Erdoberfläche. Sie werden hierbei in Wasser als Verteilungsmittel suspendiert oder emulgiert und mittels fein ver-

teilender Düsen gespritzt. Bei dieser Applikationsmethode beträgt die Aufwandmenge 0,5, 1,0 oder 3,0 Wirkstoff/ha. Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen angewachsen sind. Die Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform bis zu einer Wuchshöhe von 3 bis 15 cm an und behandelt sie danach. Die für die Nachauflaufanwendung benutzten Sojapflanzen zieht man in einem mit Torfmull (peat) angereichertem Substrat an. Es werden entweder direkt gesäte und in den gleichen Gefäßen aufgewachsenen Pflanzen ausgewählt, oder sie werden erst als Keimpflanzen getrennt angezogen und einige Tage vor der Behandlung in die Versuchsgefäße verpflanzt. Die Abdeckung unterbleibt bei der Nachauflaufbehandlung. Die Aufwandmengen für die Nachauflaufbehandlung variieren je nach Wirkstoff. Sie betragen 0,125, 0,25, 0,5 oder 1,0 kg Wirkstoff/ha.

Die Versuchsgefäße werden im Gewächshaus aufgestellt, wobei für wärmeliebende Arten wärmere Bereiche (20 bis 35°C) und für solche gemäßigter Klimate 10 bis 25°C bevorzugt wurden. Die Versuchsperiode erstreckt sich über 2 bis 4 Wochen. Während dieser Zeit werden die Pflanzen gepflegt, und ihre Reaktion auf die einzelnen Behandlungen ausgewertet. Bewertet wird nach einer Skala von 0 bis 100. Dabei bedeutet 0 keine Schädigung oder normaler Ablauf und 100 kein Aufgang der Pflanzen bzw. völlige Zerstörung zumindest der oberirdischen Teile.

Die in den Versuchen verwendeten Pflanzen stammen von folgenden Arten:

Abutilon theophrasti (Chinesischer Hanf), Amaranthus retroflexus (zurückgekrümmter Fuchsschwanz), Anoda cristata, Arachys hypogaea (Erdnüsse), Cassia tora, Chenopodium album (Weißer Gänsefuß), Chrysanthemum segetum (Saatwucherblume), Cyperus iria, Datura stramonium (Gemeiner Stechapfel), Daucus carota (Wilde Möhre), Euphorbia geniculata (Südamerikan. Wolfsmilch), Glycine max. (Soja), Gossypium hirsutum (Baumwolle), Ipomoea spp. (Prunkwindearten), Matricaria inodora (Duftlose Kamille), Oryza sativa (Reis), Sesbania exaltata (Turibaum), Sida spinosa, Sinapis alba (Weißer Senf), Solanum nigrum (Schwarzer Nachtschatten), Triticum aestivum (Weizen), Veronica spp. (Ehrenpreisarten), Zea mays (Mais).

Bei Nachauflaufanwendung zeigen beispielsweise die Wirkstoffe Nr. 2, 5, 13, 14, 23, 27, 34, 37, 43, 94, 97, 98 oder 110 eine gute herbizide Wirkung, insbesondere gegen unerwünschte breitblättrige Pflanzen.

Bei Vorauflaufanwendung sind beispielsweise die Wirkstoffe Nr. 4, 13, 14, 27, 43 oder 94 gut wirksam.

In Anbetracht der guten Verträglichkeit der Wirkstoffe und der Vielseitigkeit der Applikationsmethoden können die erfindungsgemäßen Verbindungen oder diese enthaltende Mittel außer bei den in den Gewächshausversuchen getesteten Nutzpflanzen noch in einer weiteren großen Zahl von Kulturpflanzen zur Beseitigung unerwünschten Pflanzenwuchses eingesetzt werden.

In Betracht kommen beispielsweise die folgenden Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum Gossypium herbaceum Gossypium vitifolium) | Baumwolle |
| Helianthus annuus | Sonnenblume |

| Botanischer Name | Deutscher Name |
|---|---|
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersiocon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- u. Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |

| Botanischer Name | Deutscher Name |
|---|---|
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium carymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Verbindungen der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem kann es von Nutzen sein, die Verbindungen der Formel I oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Es können auch nichtphytotoxische Öle und Ölkonzentrate zugesetzt werden.

Patentansprüche

1. Anilinderivate der Formel

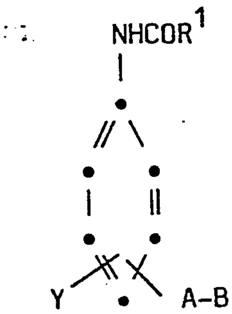

(I),

in der

$R^1$ Alkoxy, Alkylthio oder gegebenenfalls durch Halogen oder Alkoxy mit 1 bis 4 Kohlenstoffatomen substituiertes Alkyl mit jeweils bis zu 4 Kohlenstoffatomen oder Cycloalkyl mit 3 bis 6 Kohlenstoffatomen oder den Rest $-N{<}^{R^2}_{R^3}$, wobei $R^2$ und $R^3$ unabhängig voneinander Wasserstoff, Alkyl oder Alkoxy mit jeweils bis zu 4 Kohlenstoffatomen oder Cycloalkyl mit 3 bis 6 Kohlenstoffatomen oder $R^2$ und $R^3$ zusammen eine gegebenenfalls durch Methyl substituierte und gegebenenfalls Sauerstoff als Kettenglied enthaltende Alkylenkette mit bis zu 6 Kohlenstoffatomen bedeuten,

Y Wasserstoff, Halogen, Methyl, Methoxy oder Trifluormethyl,

A eine gegebenenfalls eine Ketogruppe oder eine sekundäre Carbinolgruppe als Kettenglied enthaltende, gegebenenfalls durch Methyl substituierte Alkylenkette mit 2 bis 9 Kohlenstoffatomen und

B unverzweigtes oder verzweigtes Alkyl mit bis zu 5 Kohlenstoffatomen, gegebenenfalls durch Alkyl mit 1 bis 4 Kohlenstoffatomen substituiertes

Cycloalkyl mit bis zu 8 Kchlenstoffatomen, wobei bis zu 2 Ringglieder durch Sauerstoff ersetzt sein können, oder gegebenenfalls durch Alkyl mit 1 bis 4 Kohlenstoffatomen substituiertes Cycloalkenyl mit bis zu 6 Kohlenstoffatomen bedeuten und

A und B zusammen Reste mit mindestens 7 Kohlenstoffatomen bilden.

2. Anilinderivate der Formel I gemäß Anspruch 1, dadurch gekennzeichnet, daß $R^1$ für Alkylthio oder Alkyl mit 1 bis 4 Kohlenstoffatomen, Cycloalkyl mit 3 bis 6 Kohlenstofatomen oder den Rest $-N<^{R^2}_{R^3}$ , wobei $R^2$ und $R^3$ unabhängig voneinander Wasserstoff oder Alkyl oder Alkoxy mit 1 bis 3 Kohlenstoffatomen bedeuten, Y für Wasserstoff, A für eine gegebenenfalls eine Ketogruppe als Kettenglied enthaltende Alkylenkette mit 2 bis 4 Kohlenstoffatomen und B für verzweigtes Alkyl mit 3 bis 5 Kohlenstoffatomen, für gegebenenfalls durch Alkyl mit 1 bis 3 Kohlenstoffatomen substituiertes Cycloalkyl mit 5 oder 6 Kohlenstoffatomen, wobei 2 Ringglieder durch Sauerstoff ersetzt sein können, oder für methylsubstituiertes Cyclohexenyl stehen und A und B zusammen Reste mit mindestens 7 Kohlenstoffatomen bilden.

3. N-[3-(5,5-Dimethyl-n-hexyl)-phenyl]-N'-methoxy-N'-methyl-harnstoff.

4. N-[4-(4,4-Dimethyl-n-pentan-3-on-yl)-phenyl]-N'-methoxy-N'-methyl-harnstoff.

5. Verfahren zur Herstellung von Anilinderivaten der Formel I gemäß Anspruch 1, dadurch gekennzeichnet, daß man ein Amin der Formel

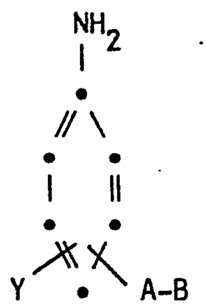

(II),

in der
Y, A und B die im Anspruch 1 genannten Bedeutungen haben,
mit einer Verbindung der Formel

$$R^1 - CO - X \qquad (III)$$

in der $R^1$ die im Anspruch 1 angegebenen Bedeutungen hat und X eine Abgangsgruppe oder den Rest $R^1$-CO-O- bedeutet, in Gegenwart eines inerten organischen Lösungsmittels und eines Säureakzeptors bei einer Temperatur im Bereich zwischen 20 und 80°C umsetzt.

6. Herbizid, enthaltend inerte Zusatzstoffe und ein Anilinderivat der Formel

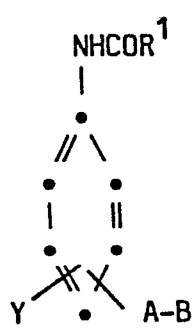

(I),

in der
$R^1$ Alkoxy, Alkylthio oder gegebenenfalls durch Halogen oder Alkoxy mit 1 bis 4 Kohlenstoffatomen substituiertes Alkyl mit jeweils bis zu 4 Kohlen-

stoffatomen oder Cycloalkyl mit 3 bis 6 Kohlenstoffatomen oder den Rest $-N{<}^{R^2}_{R^3}$, wobei $R^2$ und $R^3$ unabhängig voneinander Wasserstoff, Alkyl oder Alkoxy mit jeweils bis zu 4 Kohlenstoffatomen oder Cycloalkyl mit 3 bis 6 Kohlenstoffatomen oder $R^2$ und $R^3$ zusammen eine gegebenenfalls durch Methyl substituierte und gegebenenfalls Sauerstoff als Kettenglied enthaltende Alkylenkette mit bis zu 6 Kohlenstoffatomen bedeuten,

Y Wasserstoff, Halogen, Methyl, Methoxy oder Trifluormethyl,

A eine gegebenenfalls eine Ketogruppe oder eine sekundäre Carbinolgruppe als Kettenglied enthaltende, gegebenenfalls durch Methyl substituierte Alkylenkette mit 2 bis 9 Kohlenstoffatomen und

B unverzweigtes oder verzweigtes Alkyl mit bis zu 5 Kohlenstoffatomen, gegebenenfalls durch Alkyl mit 1 bis 4 Kohlenstoffatomen substituiertes Cycloalkyl mit bis zu 8 Kohlenstoffatomen, wobei bis zu 2 Ringglieder durch Sauerstoff ersetzt sein können, oder gegebenenfalls durch Alkyl mit 1 bis 4 Kohlenstoffatomen substituiertes Cycloalkenyl mit bis zu 6 Kohlenstoffatomen bedeuten und

A und B zusammen Reste mit mindestens 7 Kohlenstoffatomen bilden.

7. Herbizid, enthaltend inerte Zusatzstoffe und ein Anilinderivat der Formel I gemäß Anspruch 1, wobei $R^1$ für Alkylthio oder Alkyl mit 1 bis 4 Kohlenstoffatomen, Cycloalkyl mit 3 bis 6 Kohlenstofatomen oder den Rest $-N{<}^{R^2}_{R^3}$ , wobei $R^2$ und $R^3$ unabhängig voneinander Wasserstoff oder Alkyl oder Alkoxy mit 1 bis 3 Kohlenstoffatomen bedeuten, Y für Wasserstoff, A für eine gegebenenfalls eine Ketogruppe als Kettenglied enthaltende Alkylenkette mit 2 bis

4 Kohlenstoffatomen und B für verzweigtes Alkyl mit 3 bis 5 Kohlenstoffatomen, für gegebenenfalls durch Alkyl mit 1 bis 3 Kohlenstoffatomen substituiertes Cycloalkyl mit 5 oder 6 Kohlenstoffatomen, wobei 2 Ringglieder durch Sauerstoff ersetzt sein können, oder für methylsubstituiertes Cyclohexenyl stehen und A und B zusammen Reste mit mindestens 7 Kohlenstoffatomen bilden.

8. Verfahren zur Bekämpfung unerwünschten Pflanzenwuchses, dadurch gekennzeichnet, daß man eine herbizid wirksame Menge eines Anilinderivats der Formel I gemäß Anspruch 1 auf die Pflanzen und/oder ihren Lebensraum einwirken läßt.

9. Amine der Formel

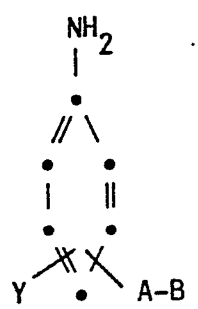

(II),

in der

Y Wasserstoff, Halogen, Methyl, Methoxy oder Trifluormethyl,

A eine gegebenenfalls eine Ketogruppe oder eine sekundäre Carbinolgruppe als Kettenglied enthaltende, gegebenenfalls durch Methyl substituierte Alkylenkette mit 2 bis 9 Kohlenstoffatomen und

B unverzweigtes oder verzweigtes Alkyl mit bis zu 4 Kohlenstoffatomen, gegebenenfalls durch Alkyl mit 1 bis 4 Kohlenstoffatomen substituiertes Cycloalkyl mit bis zu 6 Kohlenstoffatomen, wobei bis zu 2 Ringglieder durch Sauerstoff ersetzt

sein können, oder gegebenenfalls durch Alkyl mit 1 bis 4 Kohlenstoffatomen substituiertes Cycloalkenyl mit bis zu 6 Kohlenstoffatomen bedeuten und

A und B zusammen Reste mit mindestens 7 Kohlenstoffatomen bilden.

10. Verwendung von Aminen der Formel II gemäß Anspruch 9 zur Herstellung von Anilinderivaten der Formel I gemäß Anspruch 1.